(19) Europäisches Patentamt
European Patent Office
Office européen des brevets

(11) Numéro de publication : 0 392 883 B1

(12) # FASCICULE DE BREVET EUROPEEN

(45) Date de publication du fascicule du brevet : **28.04.93 Bulletin 93/17**

(51) Int. Cl.$^5$ : **A61K 7/42,** A61K 7/00, A61K 7/48

(21) Numéro de dépôt : **90400346.4**

(22) Date de dépôt : **08.02.90**

(54) **Utilisation et compositions cosmétiques contenant des diorganopolysiloxanes à fonction benzotriazole pour la protection de la peau et des cheveux.**

(30) Priorité : **15.02.89 FR 8901990**

(43) Date de publication de la demande : **17.10.90 Bulletin 90/42**

(45) Mention de la délivrance du brevet : **28.04.93 Bulletin 93/17**

(84) Etats contractants désignés : **AT BE CH DE DK ES FR GB GR IT LI NL SE**

(56) Documents cités :
**EP-A- 0 138 321**
**EP-A- 0 354 145**
**FR-A- 2 601 365**
**GB-A- 2 077 280**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lang, Gérard**
**44 avenue Lacour**
**F-95210 Saint-Gratien (FR)**
Inventeur : **Richard, Hervé**
**48 rue de l'Ermitage**
**F-75020 Paris (FR)**
Inventeur : **Grognet, Jean-Claude**
**Les Attelages du Moulin**
**F-60300 Montlognon Senlis (FR)**

(74) Mandataire : **Casalonga, Axel et al**
**BUREAU D.A. CASALONGA - JOSSE**
**Morassistrasse 8**
**W-8000 München 5 (DE)**

EP 0 392 883 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à l'utilisation en cosmétique, notamment en tant qu'agents filtrant le rayonnement UV, de diorganopolysiloxanes à fonction benzotriazole ainsi qu'aux nouvelles compositions cosmétiques contenant ces composés, destinées à la protection de la peau et des cheveux.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

Il est également souhaitable d'assurer aux cheveux une bonne protection contre la dégradation photochimique afin d'éviter en particulier une décoloration ou un changement de nuance.

On sait, par ailleurs, greffer sur des chaînes de polymères carbonés synthétiques, de polymères naturels, d'hydrolysats de protéines ou de polyaminoamides, des restes de molécules ayant un effet filtre vis-à-vis du rayonnement UV; ces polymères greffés décrits par exemple dans les brevets français n° 2 197 023, 2 237 912, 2 531 960, 2 548 018, 2 549 069, 2 586 692 et 2 586 693 peuvent être utilisés pour préparer des compositions cosmétiques protectrices de l'épiderme humain ou anti-solaires. On a cependant constaté que ces polymères greffés sont généralement peu solubles dans les solvants cosmétiques usuels, notamment dans les supports gras, et qu'ils forment des films dont la structure est trop rigide.

Or, la demanderesse a découvert que certains diorganopolysiloxanes à fonction benzotriazole présentaient, de manière étonnante, de bonnes propriétés cosmétiques associées à de bonnes propriétés filtrantes dans une large gamme de longueurs d'onde allant de 280 à 360 nm. Ils présentent notamment un excellent caractère liposoluble, ce qui les rend utilisables dans les supports gras utilisés en cosmétique. Outre leur bon pouvoir filtrant et leur bonne solubilité dans les corps gras et les solvants cosmétiques usuels, ces diorganopolysiloxanes à fonction benzotriazole présentent une excellente stabilité chimique et photochimique et ont l'avantage d'apporter de la douceur à la peau et aux cheveux, par lesquels ils sont bien tolérés.

La présente invention a donc pour objet l'utilisation en cosmétique, en particulier en tant qu'agents filtrant le rayonnement UV de longueurs d'onde comprises entre 280 et 360 nm, de diorganopolysiloxanes à fonction benzotriazole choisis parmi ceux de formule :

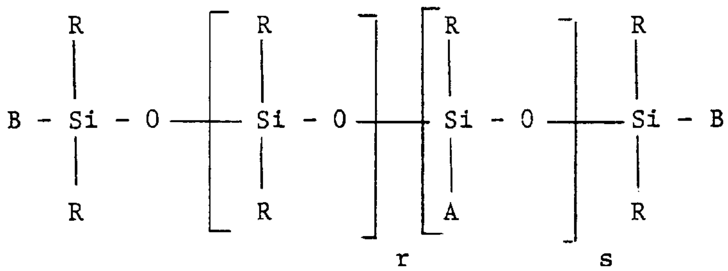

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A, et ceux de formule :

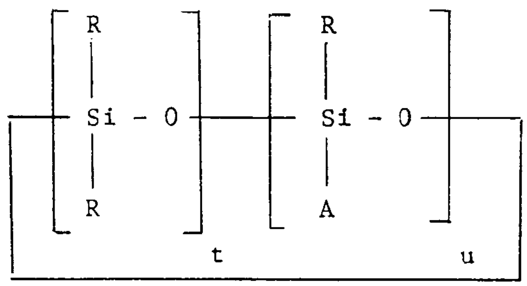
(2)

dans laquelle

R a la même signification qu'à la formule (1),

u est un nombre compris entre 1 et 20 inclus et

t est un nombre compris entre 0 et 20 inclus,

t + u est égal ou supérieur à 3,

formules dans lesquelles le symbole A est un radical de formule :

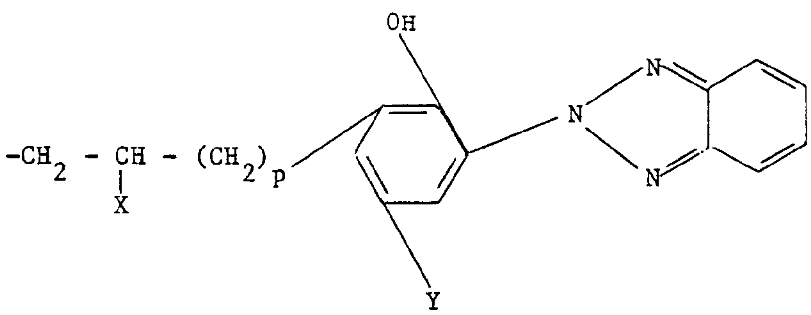
(3)

dans laquelle :

- X représente un atome d'hydrogène ou un radical alkyle en $C_1$-$C_4$
- p représente un nombre entier compris entre 1 et 10
- Y représente un atome d'hydrogène ou un radical alkyle en $C_1$-$C_8$.

Dans les formules ci-dessus, les radicaux alkyle peuvent être linéaires ou ramifiés et choisis notamment parmi les radicaux méthyle, éthyle, n-propyle, isopropyle, n-butyle, isobutyle, tert.-butyle, n-amyle, isoamyle, néopentyle, n-hexyle, n-heptyle, n-octyle, éthyl-2 hexyle et tert.-octyle.

Les radicaux R alkyle préférés sont méthyle, éthyle, propyle, n-butyle, n-octyle et éthyl-2 hexyle.

On préfère plus particulièrement les polymères statistiques où à blocs de formules (1) et (2) présentant au moins l'une des caractéristiques suivantes :

- R est méthyle
- B est méthyle
- Y est méthyle
- p = 1
- X est un atome d'hydrogène ou un radical méthyle
- r est compris entre 5 et 20 inclus
- s est compris entre 2 et 15 inclus
- t + u est compris entre 3 et 10 inclus.

Pour préparer les polymères de formules (1) et (2), on peut par exemple partir du polymère correspondant dans lequel tous les radicaux A sont des atomes d'hydrogène.

Ce polymère est dénommé par la suite polymère à SiH; les groupes SiH peuvent être présents dans la chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3 220 972, US-A-3 436 366, US-A-3 697 473 et US-A-4 340 709.

Ce polymère à SiH peut donc être choisi parmi ceux de formule :

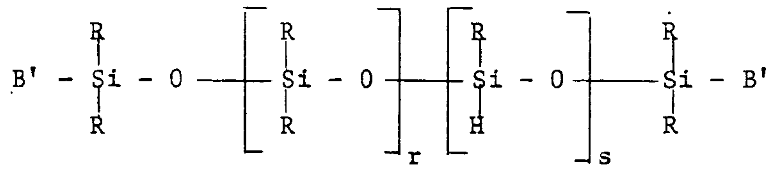

(4)

dans laquelle R, r et s ont la signification donnée ci-dessus pour la formule (1) et les radicaux B', identiques ou différents, sont choisis parmi les radicaux R et un atome d'hydrogène, et de formule :

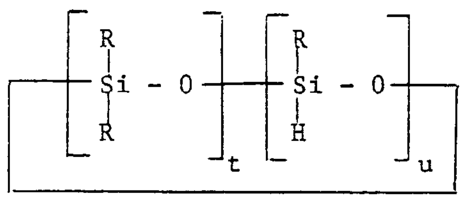

(5)

dans laquelle R, t et u ont la signification donnée ci-dessus pour la formule (2).

Sur ce polymère à SiH de formule (4) ou (5), on effectue une réaction d'hydrosilylation en présence d'une quantité catalytiquement efficace d'un catalyseur au platine sur un dérivé organique de benzotriazole choisi parmi ceux de formule :

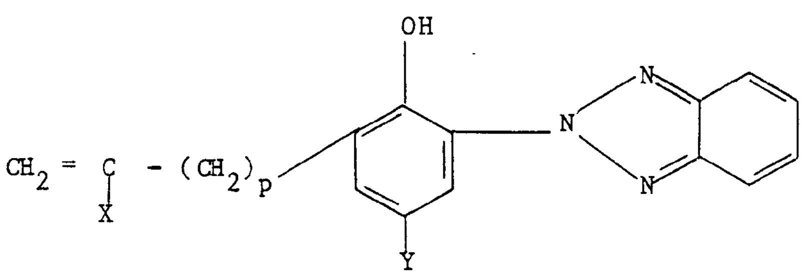

(6)

dans laquelle X, Y et p ont la même signification que ci-dessus.

Les produits de formule (6) et leur procédé de préparation sont notamment décrits dans les brevets US-4 316 033 et 4 373 060.

Le procédé recommandé s'effectue en deux étapes. Au cours de la première étape on fait réagir un halogénure d'alcényle de formule :

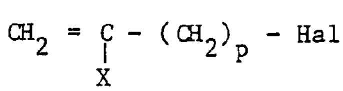

(7)

sur un benzotriazole de formule :

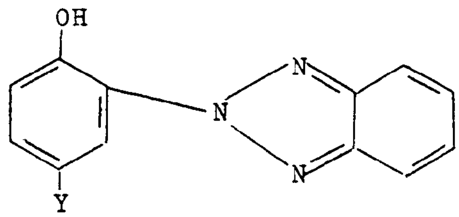

(8)

formules dans lesquelles X, Y et p ont la même signification que ci-dessus et Hal. représente un halogène, de préférence le chlore ou le brome.

Cette première étape est effectuée en présence d'une base par exemple en présence d'un hydroxyde ou carbonate de métal alcalin ou alcalino-terreux ou d'un amidure, alcoolate ou hydrure alcalin, dans un solvant compatible avec la nature de la base tel que l'eau ou un solvant organique tel qu'un alcool, le dioxane, le diméthylsulfoxyde ou le diméthylformamide, à une température comprise entre la température ambiante et le point d'ébullition du solvant, et l'on obtient le produit de formule :

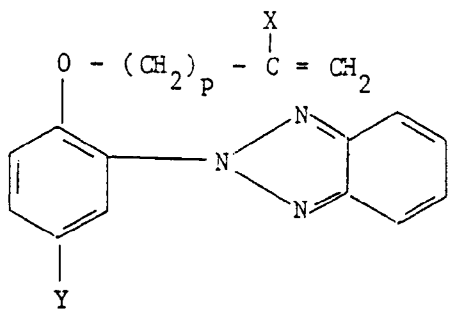

(9)

Sur le composé de formule (9), où X, Y et p ont la même signification que ci-dessus, on effectue un réarrangement de CLAISEN pour obtenir le produit désiré de formule (6).

Le réarrangement de CLAISEN peut être effectué dans les conditions décrites par TARBELL (Organic réactions, Vol. 2, John WILEY, New-York, 1944, page 1), par chauffage à au moins 170°C environ du composé de formule (9), éventuellement en présence d'un solvant.

Les catalyseurs au platine utilisés pour réaliser la réaction d'hydrosilylation des polymères de formule (4) ou (5) sur le dérivé organique de formule (6) sont amplement décrits dans la littérature. On peut en particulier citer les complexes du platine et d'un produit organique décrit dans les brevets américains US-A-3 159 601, US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A 3 377 432 et US-A-3 814 730.

Pour faire réagir le polymère à SiH de formule (4) ou (5) sur le dérivé de formule (6), on utilise généralement une quantité de catalyseur au platine, calculée en poids de platine métal, comprise entre 5 et 600 ppm, de préférence entre 10 et 200 ppm, basée sur le poids de polymère à SiH de formule (4) ou (5).

La réaction d'hydrosilylation peut avoir lieu en masse ou au sein d'un solvant organique volatil tel que le toluène, l'heptane, le xylène, le tétrahydrofuranne et le tétrachloréthylène.

Il est généralement souhaitable de chauffer le mélange réactionnel à une température de 60 à 120°C pendant le temps nécessaire pour que la réaction soit complète. Par ailleurs, on peut ajouter goutte à goutte le polymère à SiH sur le dérivé de formule (6) en solution dans un solvant organique contenant le catalyseur. On peut aussi ajouter simultanément le polymère à SiH et le dérivé de formule (6) à une suspension de catalyseur dans un solvant organique.

On vérifie que la réaction est complète en dosant les SiH résiduels par la potasse alcoolique, puis on élimine le solvant, par exemple par distillation sous pression réduite.

L'huile brute obtenue peut être purifiée, par exemple par passage sur une colonne absorbante de silice.

Un autre objet de l'invention est constitué par les compositions cosmétiques destinées à protéger la peau et les cheveux du rayonnement UV, contenant une quantité efficace d'un diorganopolysiloxane à fonction benzotriazole de formule (1) ou (2), dans un milieu cosmétiquement acceptable.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (1) ou (2) contenu dans un support cosmétiquement acceptable

comprenant au moins une phase grasse.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un diorganopolysiloxane à fonction benzotriazole de formule (1) ou (2) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses, alcooliques ou oléoalcooliques, d'émulsions telles qu'une crème ou un lait, de gels oléoalcooliques, alcooliques ou hydroalcooliques, de bâtonnets solides, ou être conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (1) ou (2) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (1) ou (2), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (1) ou (2) et pouvant contenir d'autres filtres UV-B et/ou UV-A.

Dans ce cas, la quantité totale de filtres présents dans la composition anti-solaire, c'est-à-dire le composé de formule (1) ou (2) et éventuellement les autres filtres, est comprise entre 0,5 et 15% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de spray de coiffage, de laque pour cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 5% en poids de composé de formule (1) ou (2).

La présente invention vise également les compositions cosmétiques contenant au moins un composé de formule (1) ou (2) à titre d'agent de protection contre les rayons ultraviolets constituées par des compositions

capillaires tels que les laques pour cheveux, les lotions de mise en plis éventuellement traitantes ou démêlantes, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (1) ou (2).

L'invention vise également un procédé de protection des compositions cosmétiques contre les rayons ultraviolets, consistant à incorporer à ces compositions une quantité efficace d'au moins un composé de formule (1) ou (2).

Les exemples ci-après illustrent l'invention sans en limiter la portée.

EXEMPLE 1

Préparation du polymère statistique de formule :

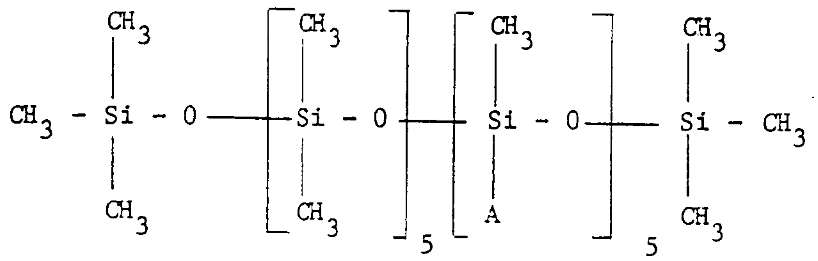

(1)

dans laquelle A représente :

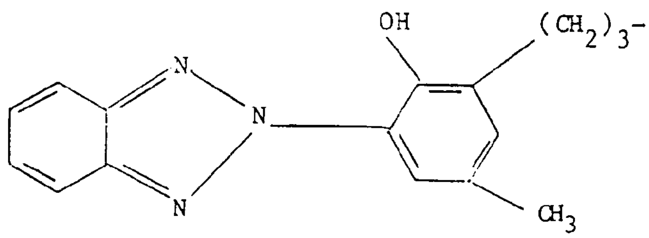

A une suspension de platine sur charbon à 5% (70 mg) dans du toluène sec (5 ml) à 90-100°C sous azote et sous agitation, on ajoute goutte à goutte en 1 heure 30 minutes, une solution dans du toluène (40 ml) de 17,1 g d allyl-3 hydroxy-2 méthyl-5 phénylbenzotriazole et 10,5 g du polymère statistique de formule ci-dessus où A est un atome d'hydrogène, tout en maintenant la température entre 100 et 105°C.

On laisse sous agitation et au reflux jusqu'à disparition des groupements SiH (absence de bande à 218ocm$^{-1}$ en infrarouge), soit 10 heures. On filtre sur papier, on élimine le solvant et on lave trois fois à l'éthanol à 80%. L'huile obtenue est reprise dans le chloroforme, séchée sur sulfate de sodium et filtrée sur célite pour éliminer les restes de platine colloïdal. On obtient après évaporation du solvant une huile jaune orange (poids : 26 g, rendement : 95%).

Spectre UV ($CHCl_3$) : $\lambda max_1$ : 307 nm.
$\lambda max_2$ : 345 nm.

L'analyse par résonance magnétique nucléaire ($^1H$ et $^{29}Si$ RMN) indique que le produit a bien la structure souhaitée.

EXEMPLE 2

Dans un ballon tricol de 100 ml maintenu à 110°C par un bain d'huile, muni d'une agitation magnétique et d'un réfrigérant ascendant, on charge 18,8 g (0,071 mole) d'allyl-3 hydroxy-2 méthyl-5 phénylbenzotriazole, 25 ml de toluène et 6µl d'une solution dans l'hexane (à 8,45% en poids de platine métal) d'un complexe de

platine préparé à partir d'acide chloroplatinique et de divinyl-1,3 tétraméthyl-1,1,3,3 disiloxane comme décrit dans le brevet US-A-3 814 730.

On ajoute en deux heures 10 g d'un copolymère statistique à SiH de formule :

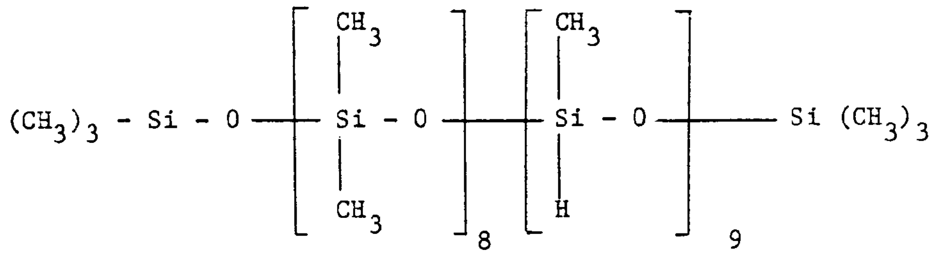

titrant 713 meq/100 g en fonction SiH (meq = milli-équivalent).

Au bout de 7 heures de réaction on détermine, par dosage des SiH résiduels au moyen de potasse butanolique, que le taux de transformation des fonctions SiH est de 88%.

On obtient alors une huile limpide, de couleur orangée de très forte viscosité après avoir éliminé le toluène par distillation à 110°C sous pression réduite de 3,3 KPa.

Spectre UV ($CHCl_3$) $\lambda\ max_1$ = 307 nm
$\lambda\ max_2$ = 345 nm

L'analyse par résonance magnétique nucléaire ($^1H$) indique que le produit a bien la structure souhaitée.

## EXEMPLE 3

On effectue exactement les mêmes opérations qu'à l'exemple 2 sauf que l'on utilise un rapport molaire allyl-3 hydroxy-2 méthyl-5 phénylbenzotriazole/SiH égal à 1,3; à savoir :

- 24,5 g d'allyl-3 hydroxy-2 méthyl-5 phénylbenzotriazole pour 10 g de polymère à SiH.

Après 7 heures de réaction, le taux de transformation des fonctions SiH est de 98%. On obtient après élimination du toluène à 60°C sous pression réduite de 0,6 KPa, 32,5 g d'une huile très visqueuse de couleur jaune orange contenant 16% en poids de monomères résiduels.

L'élimination de ces monomères est effectuée par passage de l'huile sur une colonne de gel de silice (support Kieselgel ART 7754, MERCK) avec comme solvant d'élution des monomères, un mélange 40/60 en volume de dichlorométhane/ heptane et comme solvant d'élution de l'huile, l'acétate d'éthyle. Après élimination de l'acétate d'éthyle, on obtient une gomme translucide de coloration orange contenant 65% en poids de produit attendu.

Spectre UV ($CHCl_3$) = $\lambda\ max_1$ = 307 nm
$\lambda\ max_2$ = 345 nm.

## EXEMPLES D'APPLICATION

### Exemple A : Emulsion huile-dans-l'eau antisolaire

| | | |
|---|---|---|
| - Composé de l'exemple 1 | | 3,0 g |
| - Alcool cétylstéarylique oxyéthyléné (C16/C18 - 35 65) (15 OE) ("MERGITAL CS 15" vendu par la Société HENKEL) | | 3,0 g |
| - Monostéarate de glycérol | | 4,8 g |
| - Alcool myristique | | 4,5 g |
| - Benzoate d'alcools C12/C15 ("FINSOLV TN" vendu par la Société WITCO) | | 18,0 g |
| - Propylène glycol | | 6,0 g |
| - Conservateur | | 0,2 g |
| - Parfum | | 0,6 g |
| - Eau déminéralisée | qsp | 100 g |

On chauffe les corps gras et les émulsionnants vers 80-85°C; on ajoute le composé de l'exemple 1. Par ailleurs, on chauffe à 80-85°C l'eau contenant les composés hydrosolubles et on ajoute la phase grasse à la phase aqueuse. Après 10 minutes d'agitation vive, on laisse refroidir sous agitation modérée, puis on ajoute le parfum et le conservateur.

### Exemple B : Stick antisolaire

| | | |
|---|---|---|
| - Composé de l'exemple 1 | | 3,0 g |
| - Cire minérale d'hydrocarbure | | 20,0 g |
| - Cire d'abeilles | | 7,0 g |
| - Alcool oléique | | 12,0 g |
| - Lanoline hydrogénée | | 8,0 g |
| - Lanoline liquide | | 8,0 g |
| - Cire de carnauba | | 1,0 g |
| - Benzoate d'alcools C12/C15 ("FINSOLV TN" vendu par la Société WITCO) | | 20,0 g |
| - Parfum | | 1,2 g |
| - Huile de vaseline | qsp | 100 g |

Exemple C : Emulsion huile-dans-l'eau protectrice de l'épiderme humain

| | | | |
|---|---|---|---|
| - Composé de l'exemple 2 | | 2 | g |
| - Alcool cétylstéarylique oxyéthyléné à 15 moles d'O.E. | | 3 | g |
| - Monostéarate de glycérol | | 4,8 | g |
| - Alcool myristique | | 4,5 | g |
| - Benzoate d'alcools $C_{12}/C_{15}$ | | 18,0 | g |
| - Propylène glycol | | 6,0 | g |
| - Conservateur | | 0,2 | g |
| - Parfum | | 0,6 | g |
| - Eau déminéralisée | qsp | 100 | g |

Cette émulsion est préparée comme dans l'exemple A.

Exemple D : Stick antisolaire

| | | | |
|---|---|---|---|
| - Composé de l'exemple 3 | | 2 | g |
| - Composé de l'exemple 2 | | 1 | g |
| - Cire minérale d'hydrocarbure | | 20 | g |
| - Cire d'abeille | | 7 | g |
| - Alcool oléique | | 12 | g |
| - Lanoline hydrogénée | | 8 | g |
| - Lanoline liquide | | 8 | g |
| - Cire de carnauba | | 1 | g |
| - Benzoate d'alcools $C_{12}/C_{15}$ | | 20 | g |
| - Parfum | qs | | |
| - Huile de vaseline | qsp | 100 | g |

Exemple E : Crème antisolaire

| | | |
|---|---|---|
| - Composé de l'exemple 3 | | 5 g |
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | | 7 g |
| - Mélange de mono et distéarate de glycérol non autoémulsionnable | | 2 g |
| - Alcool cétylique | | 1,5 g |
| - Benzoate d'alcools en $C_{12}$-$C_{15}$ vendu par la Société WITCO sous le dénomination "FINSOLV TN" | | 15 g |
| - Polydiméthylsiloxane | | 1,5 g |
| - Glycérine | | 20 g |
| - Parfum, conservateur | qs | |
| - Parfum, conservateur | qsp | 100 g |

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée et vers 40°C, on ajoute parfum et conservateur.

Les exemples précédents A à E illustrent des compositions destinées à être appliquées sur la peau pour la protéger des rayons UV.

Les exemples suivants illustrent des compositions capillaires.

Exemple F : Lotion protectrice des cheveux

| | | |
|---|---|---|
| - Composé de l'exemple 1 | | 1,4 g |
| - Benzoate d'alcools $C_{12}$/$C_{15}$ vendu sous le nom de "FINSOLV TN" par la Société WITCO | qsp | 100 g |

Cette solution limpide appliquée sur cheveux mouillés apporte de la brillance, de la douceur et du gonflant aux cheveux séchés tout en les protégeant du soleil.

Exemple G : Lotion protectrice des cheveux

| | | |
|---|---|---|
| - Composé de l'exemple 2 | | 5 g |
| - Alcool éthylique absolu | | 10 g |
| - Triglycérides d'acides gras en $C_8$-$C_{12}$ vendus sous le nom de "MIGLYOL 812" par la Société DYNAMIT NOBEL | qsp | 100 g |

Cette lotion a l'aspect d'un liquide limpide légèrement ambré.

Appliquée sur cheveux mouillés préalablement lavés et rincés, cette lotion permet un coiffage plus facile tout en protégeant les cheveux séchés du rayonnement ultra-violet.

**Revendications**

1. Utilisation en cosmétique de diorganopolysiloxanes à fonction benzotriazole choisis parmi ceux de formule :

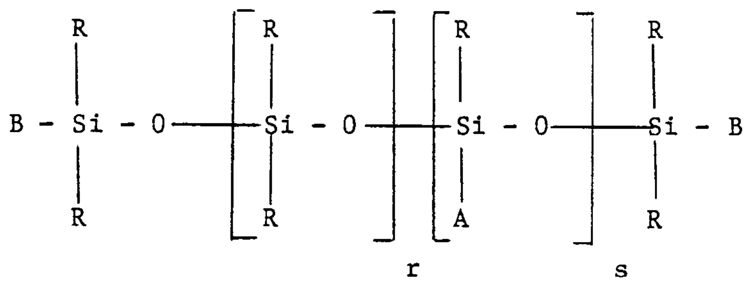

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

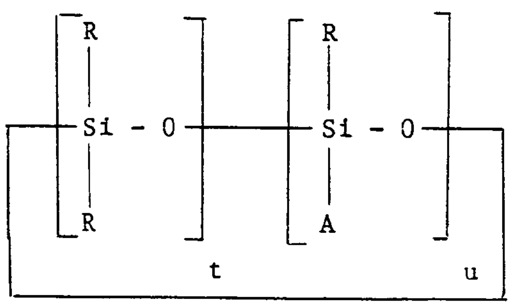

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

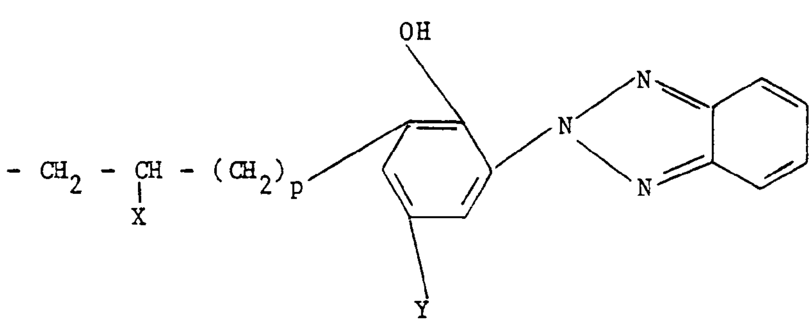

(3)

dans laquelle :

X représente un atome d'hydrogène ou un radical alkyle en $C_1$-$C_4$,
p représente un nombre entier compris entre 1 et 10,
Y représente un atome d'hydrogène ou un radical alkyle en $C_1$-$C_8$.

**2.** Utilisation en cosmétique d'un polymère statistique ou à blocs de formule (1) ou (2) selon la revendication 1, présentant au moins l'une des caractéristiques suivantes :

- R est méthyle,
- B est méthyle,
- Y est méthyle
- p = 1
- X est un atome d'hydrogène ou méthyle
- r est compris entre 5 et 20 inclus
- s est compris entre 2 et 15 inclus
- t + u est compris entre 3 et 10 inclus.

**3.** Utilisation en cosmétique à titre d'agents filtrant le rayonnement UV de longueurs d'onde comprises entre 280 et 360 nm, de diorganopolysiloxanes à fonction benzotriazole choisis parmi ceux de formule :

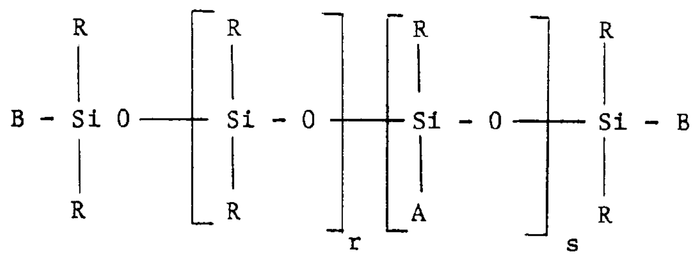

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

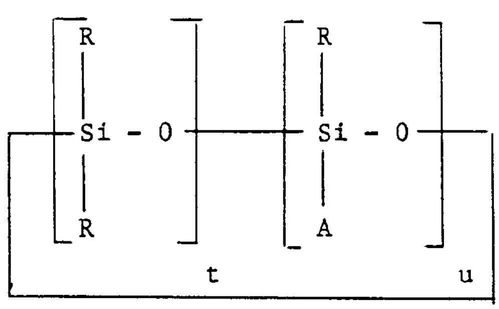

(2)

dans laquelle

R a la même signification qu'à la formule (1),
u est un nombre compris entre 1 et 20 inclus,
t est un nombre compris entre 0 et 20 inclus,
t + u est égal ou supérieur à 3,

formules dans lesquelles le symbole A est un radical de formule :

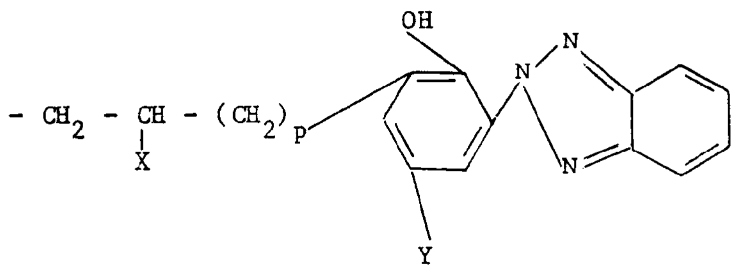

(3)

dans laquelle :

X représente un atome d'hydrogène ou un radical alkyle en $C_1$-$C_4$,

p représente un nombre entier compris entre 1 et 10,

Y représente un atome d'hydrogène ou un radical alkyle en $C_1$-$C_8$.

**4.** Utilisation en cosmétique à titre d'agents filtrant le rayonnement UV de longueurs d'onde comprises entre 280 et 360 nm, de diorganopolysiloxanes statistiques ou à blocs de formule (1) ou (2) selon la revendication 3, présentant au moins l'une des caractéristiques suivantes :

R est méthyle

B est méthyle

Y est méthyle

p = 1

X est un atome d'hydrogène ou méthyle

r est compris entre 5 et 20 inclus

s est compris entre 2 et 15 inclus

t + u est compris entre 3 et 10 inclus.

**5.** Utilisation en cosmétique à titre d'agents filtrant le rayonnement UV de longueurs d'onde comprises entre 280 et 360 nm, d'un polydiméthylsiloxane à greffons allyl-3 hydroxy-2 méthyl-5 phénylbenzotriazole de formule (1) selon la revendication 1 dans laquelle R et B désignent méthyle, r = 5 et s = 5.

**6.** Utilisation en cosmétique à titre d'agent filtrant le rayonnement UV de longueurs d'onde comprises entre 280 et 360 nm d'un polydiméthylsiloxane à greffons allyl-3 hydroxy-2 méthyl-5 phénylbenzotriazole de formule (1) selon la revendication 1, dans laquelle R et B désignent méthyle r = 8 et s = 9.

**7.** Composition cosmétique, caractérisée par le fait qu'elle comprend, dans un support cosmétiquement acceptable, une quantité efficace d'au moins un diorganopolysiloxane à fonction benzotriazole choisi parmi ceux de formule :

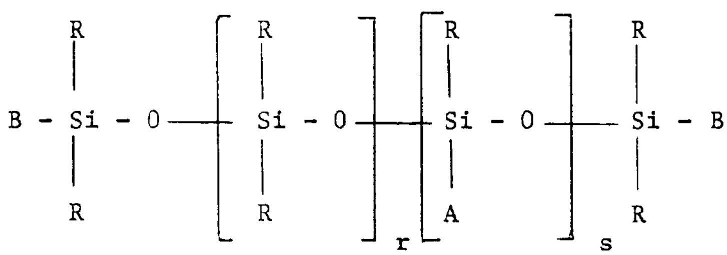

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A, et ceux de formule :

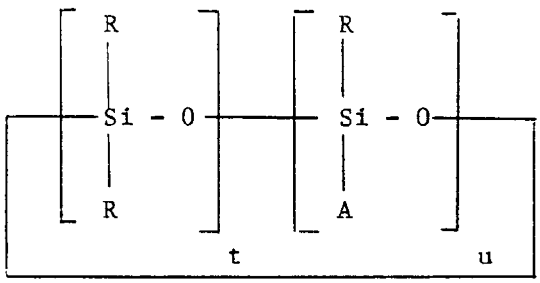

(2)

dans laquelle
R a la même signification qu'à la formule (1),
u est un nombre compris entre 1 et 20 inclus,
t est un nombre compris entre 0 et 20 inclus,
t + u est égal ou supérieur à 3,
formules dans lesquelles le symbole A est un radical de formule :

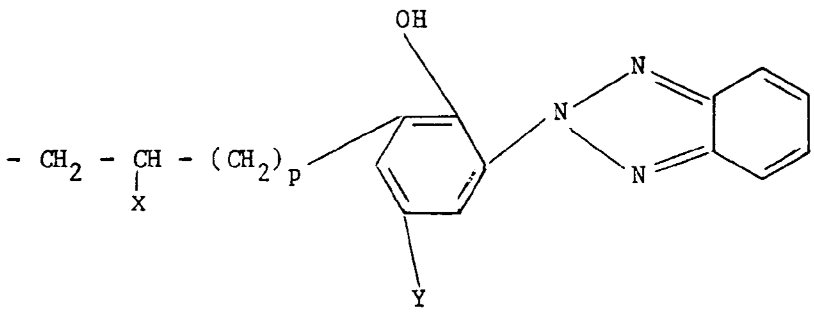

(3)

dans laquelle :
X représente un atome d'hydrogène ou un radical alkyle en $C_1$-$C_4$,
p représente un nombre entier compris entre 1 et 10,
Y représente un atome d'hydrogène ou un radical alkyle en $C_1$-$C_8$.

**8.** Composition cosmétique selon la revendication 7, caractérisée par le fait qu'elle comprend un diorganopolysiloxane à fonction benzotriazole de formule (1) ou (2), statistique ou à blocs, présentant au moins l'une des caractéristiques suivantes : R est méthyle, B est méthyle, r est compris entre 5 et 20 inclus, s est compris entre 2 et 15 inclus, t + u est compris entre 3 et 10 inclus, X est H ou méthyle, p = 1 et Y est méthyle.

**9.** Composition cosmétique selon la revendication 7 ou 8, caractérisée par le fait qu'elle comprend un polydiméthylsiloxane à greffons allyl-3 hydroxy-2 méthyl-5 phénylbenzotriazole de formule (1) selon la revendication 7 dans laquelle R et B désignent le groupe méthyle, r = 5 et s = 5.

**10.** Composition cosmétique selon la revendication 7 ou 8, caractérisée par le fait qu'elle comprend un polydiméthylsiloxane à greffons allyl-3 hydroxy-2 méthyl-5 phényl benzotriazole de formule (1) dans laquelle R et B désignent méthyle, r = 8 et s = 9.

**11.** Composition cosmétique selon l'une quelconque des revendications 7 à 10, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

**12.** Composition cosmétique selon l'une quelconque des revendications 7 à 11, caractérisée par le fait qu'elle se présente sous forme de lotion huileuse, alcoolique ou oléoalcoolique, d'émulsion, gel oléoalcoolique, alcoolique ou hydroalcoolique, bâtonnet solide, spray ou aérosol.

**13.** Composition cosmétique selon l'une quelconque des revendications 7 à 12, caractérisée par le fait qu'elle

constitue une composition protectrice de l'épiderme humain et contient 0,25 à 3% en poids de diorganopolysiloxane de formule (1) ou (2).

**14.** Composition cosmétique selon l'une quelconque des revendications 7 à 12, se présentant sous forme de composition anti-solaire, caractérisée par le fait qu'elle contient 0,5 à 15% en poids de diorganopolysiloxane de formule (1) ou (2).

**15.** Composition cosmétique anti-solaire selon la revendication 14, caractérisée par le fait qu'elle contient en outre un agent filtrant les rayons UV-B et/ou UV-A.

**16.** Composition cosmétique selon l'une quelconque des revendications 7 à 11, destinée à être appliquée sur les cheveux, caractérisée par le fait qu'elle se présente sous forme de shampooing, lotion, gel ou émulsion à rincer, à appliquer avant ou après le shampooing, avant ou après coloration ou décoloration, avant ou après permanente, lotion ou gel coiffants ou traitants, lotion ou gel pour le brushing ou la mise en plis, spray de coiffage, laque pour cheveux et comprend 0,25 à 5% en poids de diorganopolysiloxane de formule (1) ou (2).

**17.** Composition cosmétique selon l'une quelconque des revendications 7 à 11, se présentant sous forme d'une composition cosmétique colorée ou non, caractérisée par le fait qu'elle est constituée par une composition capillaire, un produit de maquillage ou une composition pour les soins ou le traitement de la peau, comprenant 0,25 à 3% en poids de diorganopolysiloxane de formule (1) ou (2).

**18.** Procédé cosmétique de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un diorganopolysiloxane à fonction benzotriazole de formule (1) ou (2) défini dans l'une quelconque des revendications 3 à 6.

**19.** Procédé de protection d'une composition cosmétique contre les rayons ultraviolets, caractérisé par le fait qu'il consiste à incorporer à cette composition une quantité efficace d'au moins un diorganopolysiloxane à fonction benzotriazole de formule (1) ou (2) définie dans l'une quelconque des revendications 3 à 6.

## Claims

**1.** Use in cosmetics of diorganopolysiloxanes containing a benzotriazole functional group, which are chosen from those of formula:

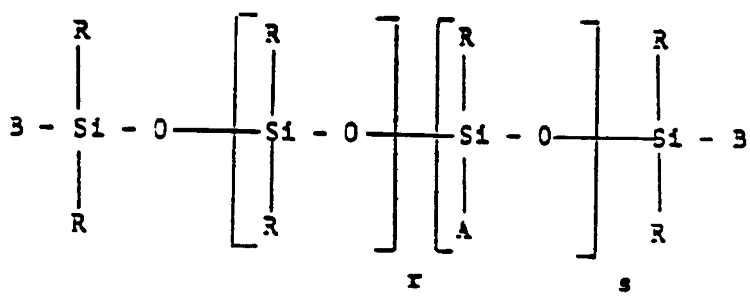

(1)

in which the symbols:

R, which are identical or different, are chosen from $C_1$-$C_{10}$ alkyl, phenyl and 3, 3, 3-trifluoropropyl radicals, at least 80 % of the number of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

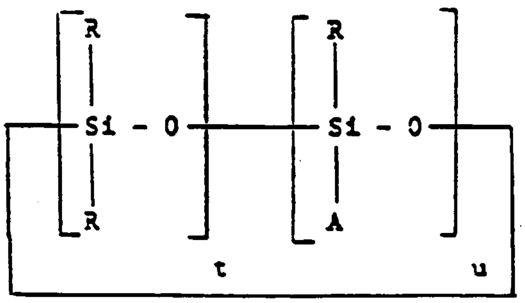

(2)

in which

R has the same meaning as in formula (1),
u is a number between 1 and 20 inclusive,
t is a number between 0 and 20 inclusive,
t + u is equal to or greater than 3,
in which formulae the symbol A is a radical of formula:

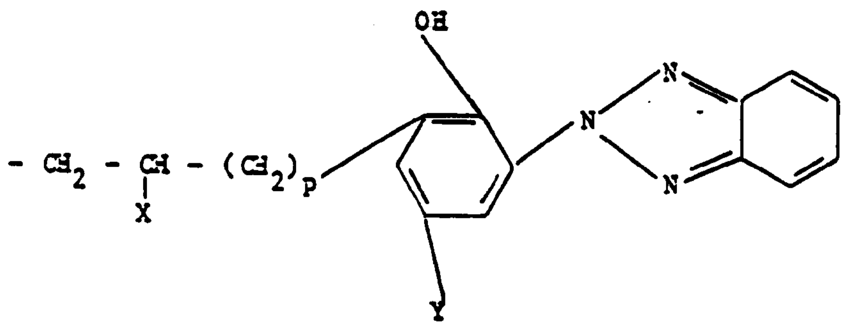

(3)

in which:

X denotes a hydrogen atom or a $C_1$-$C_4$ alkyl radical,
p denotes an integer between 1 and 10,
Y denotes a hydrogen atom or a $C_1$-$C_8$ alkyl radical.

**2.** Use in cosmetics of a random or block polymer of formula (1) or (2) according to Claim 1, exhibiting at least one of the following characteristics:

- R is methyl,
- B is methyl,
- Y is methyl
- p = 1
- X is a hydrogen atom or methyl
- r is between 5 and 20 inclusive
- s is between 2 and 15 inclusive
- t + u is between 3 and 10 inclusive.

**3.** Use in cosmetics, as agents filtering UV radiation of wavelengths between 280 and 360 nm, of diorganopolysiloxanes containing a benzotriazole functional group which are chosen from those of formula

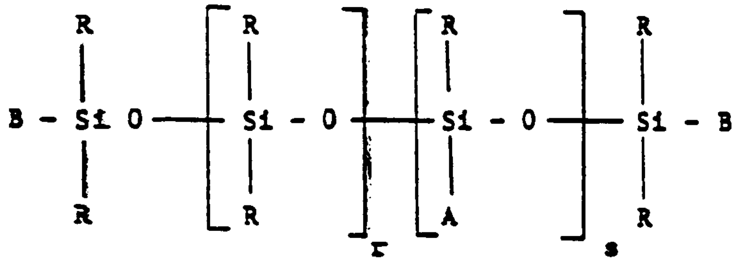

(1)

in which the symbols:

R, which are identical or different, are chosen from $C_1$-$C_{10}$ alkyl, phenyl and 3, 3, 3-trifluoropropyl radicals, at least 80 % of the number of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

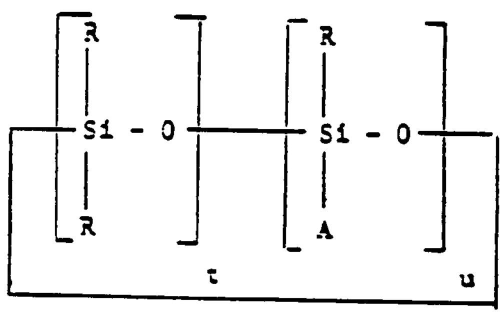

(2)

in which

R has the same meaning as in formula (1),

u is a number between 1 and 20 inclusive,

t is a number between 0 and 20 inclusive,

t + u is equal to or greater than 3,

in which formulae the symbol A is a radical of formula:

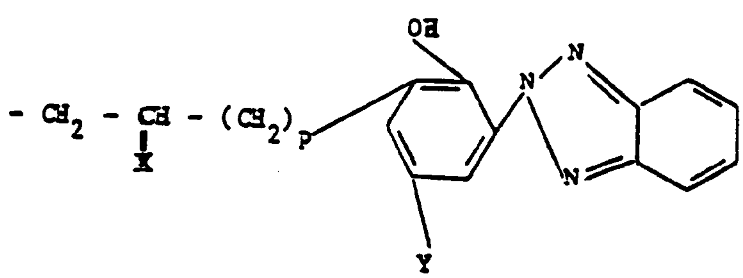

(3)

in which:

X denotes a hydrogen atom or a $C_1$-$C_4$ alkyl radical,

p denotes an integer between 1 and 10,

Y denotes a hydrogen atom or a $C_1$-$C_8$ alkyl radical.

**4.** Use in cosmetics, as agents filtering UV radiation of wavelengths between 280 and 360 nm, of random or block diorganopolysiloxanes of formula (1) or (2) according to Claim 3, exhibiting at least one of the following characteristics:

R is methyl,

B is methyl,
Y is methyl
p = 1
X is a hydrogen atom or methyl
r is between 5 and 20 inclusive
s is between 2 and 15 inclusive
t + u is between 3 and 10 inclusive.

**5.** Use in cosmetics, as agents filtering UV radiation of wavelengths between 280 and 360 nm, of a polydimethylsiloxane containing 3-allyl-2-hydroxy-5-methylphenylbenzotriazole graft units of formula (1) according to Claim 1, in which R and B denote methyl, r = 5 and s = 5.

**6.** Use in cosmetics, as agents filtering UV radiation of wavelengths between 280 and 360 nm, of a polydimethylsiloxane containing 3-allyl-2-hydroxy-5-methylphenylbenzotriazole graft units of formula (1) according to Claim 1, in which R and B denote methyl, r = 8 and s = 9.

**7.** Cosmetic composition characterised in that it comprises, in a cosmetically acceptable carrier, an effective quantity of at least one diorganopolysiloxane containing a benzotriazole functional group, which is chosen from those of formula:

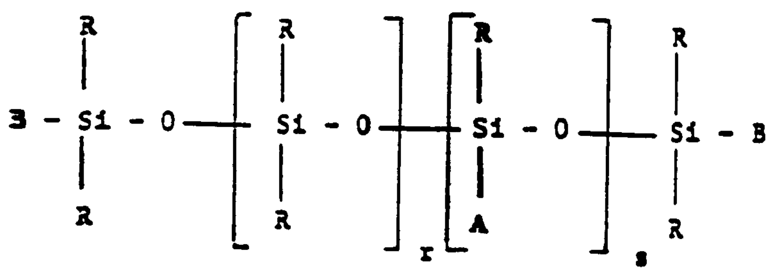

(1)

in which the symbols:

R, which are identical or different, are chosen from $C_1$-$C_{10}$ alkyl, phenyl and 3, 3, 3-trifluoropropyl radicals, at least 80 % of the number of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbyols B denotes A,

and those of formula:

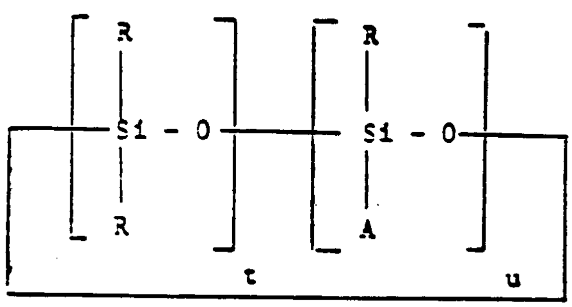

(2)

in which

R has the same meaning as in formula (1),
u is a number between 1 and 20 inclusive,
t is a number between 0 and 20 inclusive,
t + u is equal to or greater than 3,

in which formulae the symbol A is a radical of formula:

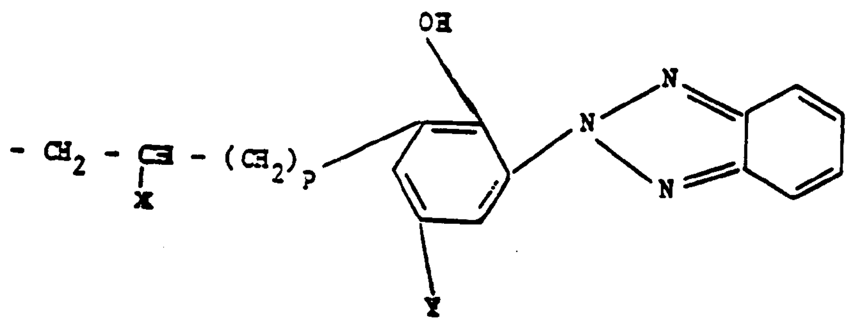

(3)

in which:

X denotes a hydrogen atom or a $C_1$-$C_4$ alkyl radical,
p denotes an integer between 1 and 10,
Y denotes a hydrogen atom or a $C_1$-$C_8$ alkyl radical.

8. Cosmetic composition according to Claim 7, characterised in that it comprises a diorganopolysiloxane containing a benzotriazole functional group of formula (1) or (2), random or containing blocks, exhibiting at least one of the following characteristics: R is methyl, B is methyl, R is between 5 and 20 inclusive, s is between 2 and 15 inclusive, t + u is between 3 and 10 inclusive, X is H or methyl, p = 1 and Y is methyl.

9. Cosmetic composition according to Claim 7 or 8, characterised in that it comprises a polydimethylsiloxane containing 3-allyl-2-hydroxy-5-methylphenylbenzotriazole graft units of formula (1) according to Claim 7, in which R and B denote the methyl group, r = 5 and s = 5.

10. Cosmetic composition according to Claim 7 or 8, characterised in that it comprises a polydimethylsiloxane containing 3-allyl-2-hydroxy-5-methylphenylbenzotriazole graft units of formula (1), in which R and B denote methyl, r = 8 and s = 9.

11. Cosmetic composition according to any one of Claims 7 to 10, characterised in that it additionally contains cosmetic adjuvants chosen from thickeners, emollients, moisteners, surfactants, preserving agents, antifoams, perfumes, oils, waxes, lanolin, lower monoalcohols and polyols, benzoates of $C_{12}$-$C_{15}$ alcohols, propellants, colorants and pigments.

12. Cosmetic composition according to any one of Claims 7 to 11, characterised in that it is in the form of an oily, alcoholic or oleoalcoholic lotion, emulsion, oleoalcoholic, alcoholic or hydroalcoholic gel, solid stick, spray or aerosol.

13. Cosmetic composition according to any one of Claims 7 to 12, characterised in that it constitutes a protective composition for the human epidermis and contains 0.25 to 3 % by weight of diorganopolysiloxane of formula (1) or (2).

14. Cosmetic composition according to any one of Claims 7 to 12, which is in the form of antisun composition, characterised in that it contains 0.5 to 15 % by weight of diorganopolysiloxane of formula (1) or (2).

15. Antisun cosmetic composition according to Claim 14, characterised in that it additionally contains a sunscreen for UV-B and/or UV-A rays.

16. Cosmetic composition according to any one of Claims 7 to 11, intended to be applied to hair, characterised in that it is in the form of shampoo, lotion, gel or emulsion for rinsing, to be applied before or after shampooing, before or after dyeing or bleaching, before or after permanent waving, styling or treating lotion or gel, lotion or gel for blow-drying or setting, styling spray or hair lacquer and includes 0.25 to 5% by weight of diorganopolysiloxane of formula (1) or (2).

17. Cosmetic composition according to any one of Claims 7 to 11, which is in the form of a cosmetic composition, coloured or otherwise, characterised in that it consists of a hair-care composition, a make-up product or a composition for skin care or treatment, including 0.25 to 3% by weight of diorganopolysiloxane of formula (1) or (2).

**18.** Cosmetic process for protecting the skin and hair, either natural or sensitised, against ultraviolet radiation, characterised in that is consists in applying to the skin or the hair an effective quantity of a cosmetic composition containing at least one diorganopolysiloxane containing a benzotriazole functional group of formula (1) or (2), defined in any one of Claims 3 to 6.

**19.** Process for protecting a cosmetic composition against ultraviolet rays, characterised in that it consists in incorporating into this composition an effective quantity of at least one diorganopolysiloxane containing a benzotriazole functional group of formula (1) or (2) defined in any one of Claims 3 to 6.

**Patentansprüche**

**1.** Verwendung von Diorganopolysiloxanen mit einer Benzotriazolgruppe ausgewählt unter solchen der Formel:

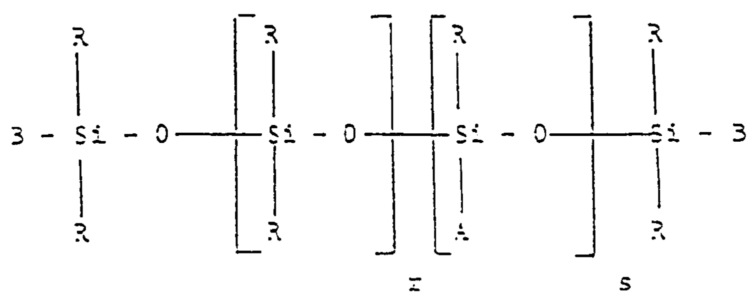

(1)

worin die Symbole bedeuten:
R ist gleich oder verschieden und ausgewählt aus den $C_1$ bis $C_{10}$-Alkylradikalen, Phenyl und 3,3,3-Trifluorpropyl, wobei mindestens 80 % der Zahl der Radikale R Methylradikale sind,
B ist gleich oder verschieden und ausgewählt aus den Radikalen R und dem Radikal A,
r ist eine Zahl ausgewählt zwischen 0 und einschließlich 200,
s ist eine Zahl ausgewählt zwischen 0 und einschließlich 50,
und wenn s 0 ist, dann bedeutet mindestens eines der beiden Symbole B A;
und solchen der Formel

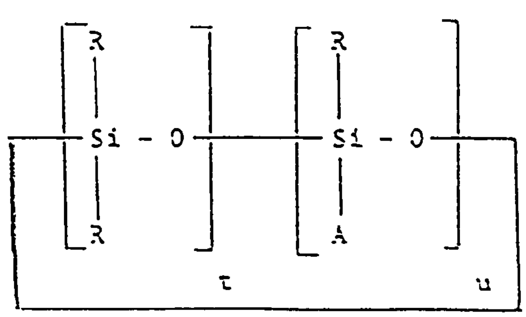

(2)

worin R die gleiche Bedeutung wie in Formel (1) besitzt,
u eine Zahl zwischen 1 und einschließlich 20 ist, und
t eine Zahl zwischen 0 und einschließlich 20 ist, und
t + u gleich oder größer 3 ist;
und in denen das Symbol A ein Radikal der Formel ist:

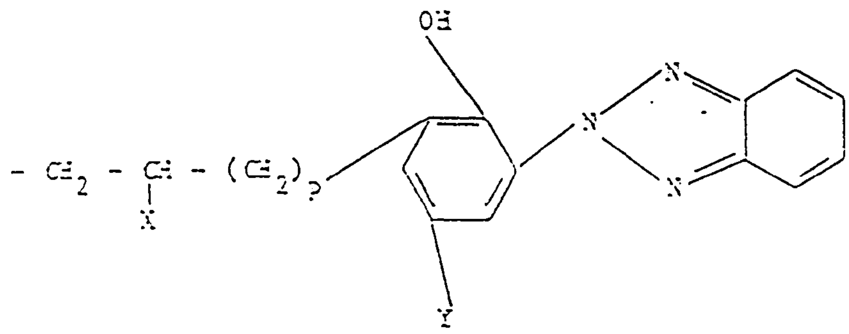

(3)

worin:

- X ein Wasserstoffatom oder ein $C_1$-$C_4$-Alkylradikal bedeutet,
- p eine ganze Zahl zwischen 1 und 10 bedeutet,
- Y ein Wasserstoffatom oder ein $C_1$-$C_8$-Alkylradikal bedeutet,

in der Kosmetik.

2. Verwendung eines statistischen Polymeren oder eines Blockpolymeren der Formel (1) oder (2) gemäß Anspruch 1 in der Kosmetik, dadurch gekennzeichnet, daß sie mindestens eine der folgenden Charakteristika besitzen:
   - R ist Methyl
   - B ist Methyl
   - Y ist Methyl
   - p = 1
   - X ist ein Wasserstoffatom oder Methyl
   - r liegt zwischen 5 und einschließlich 20
   - s liegt zwischen 2 und einschließlich 15
   - t + u liegt zwischen 3 und einschließlich 10.

3. Verwendung von Diorganopolysiloxanen mit einer Benzotriazolgruppe in der Kosmetik als Filter gegenüber UV-Strahlen der Wellenlänge zwischen 280 und 360 nm, dadurch gekennzeichnet, daß sie ausgewählt sind aus solchen der Formel:

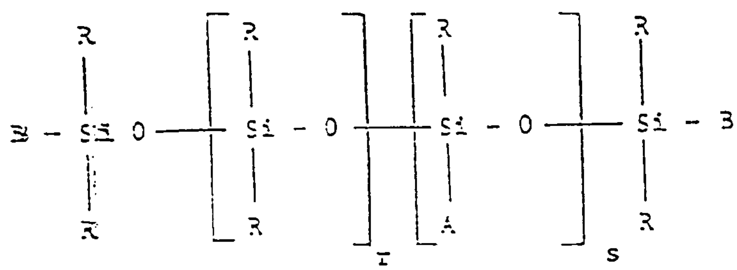

(1)

worin die Symbole bedeuten:

R ist gleich oder verschieden, und ausgewählt aus den $C_1$-$C_{10}$-Alkylradikalen, Phenyl und 3,3,3-Trifluorpropyl, und wobei mindestens 80 % der Zahl der Radikale R Methylradikale sind;

B ist identisch oder verschieden und ausgewählt aus den Radikalen R und dem Radikal A,

r ist eine Zahl ausgewählt zwischen 0 und einschließlich 200,

s ist eine Zahl ausgewählt zwischen 0 und einschließlich 50,

und wenn s 0 ist, ist mindestens eines der beiden Symbole B A, und solchen der Formel:

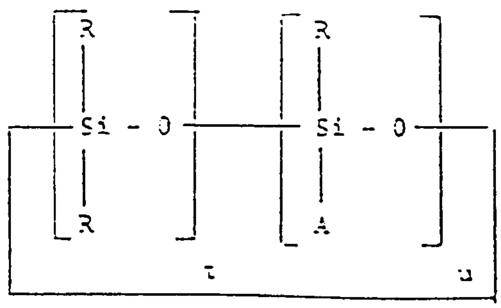

(2)

worin bedeuten:
R besitzt die gleiche Bedeutung wie in Formel (1),
u ist eine Zahl zwischen 1 und einschließlich 20,
t ist eine Zahl zwischen 0 und einschließlich 20,
t + u ist gleich oder größer als 3,
und in denen das Symbol A ein Radikal der Formel ist:

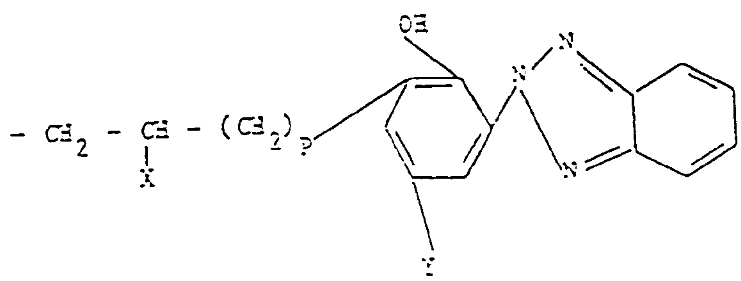

(3)

worin:
X ein Wasserstoffatom oder ein $C_1$-$C_4$-Radikal ist,
p eine ganze Zahl zwischen 1 und 10 ist,
Y ein Wasserstoffatom oder ein $C_1$-$C_8$-Radikal bedeutet.

**4.** Verwendung von statistischen oder Block-Diorganopolysiloxanen der Formel (1) oder (2) gemäß Anspruch 3 in der Kosmetik als Filter von UV-Strahlung der Wellenlänge zwischen 280 und 360 nm, dadurch gekennzeichnet, daß sie mindestens eine der folgenden Charakteristiken enthalten:
R ist Methyl
B ist Methyl
Y ist Methyl
p = 1
X ist ein Wasserstoffatom oder Methyl
r ist zwischen 5 und einschließlich 20
s ist zwischen 2 und einschließlich 15
t + u ist zwischen 3 und einschließlich 10.

**5.** Verwendung eines Polydimethylsiloxans mit Pfropfen aus 3-Allyl -2-hydroxy-5-methyl-phenyl-benzotriazol der Formel (1) gemäß Anspruch 1, worin R und B Methyl bedeuten, r = 5 und s = 5, in der Kosmetik als Filter für UV-Strahlung der Wellenlänge zwischen 280 und 360 nm.

**6.** Verwendung eines Polydimethylsiloxans mit Pfropfen aus 3-Allyl-2-hydroxy-5-methyl-phenyl-benzotriazol der Formel (1) gemäß Anspruch 1, worin R und B Methyl bedeuten, r = 8 und s = 9, in der Kosmetik als Filter für UV-Strahlung der Wellenlänge zwischen 280 und 360 nm.

**7.** Kosmetische Zusammensetzung, dadurch gekennzeichnet, daß sie in einem kosmetisch annehmbaren Träger eine wirksame Menge mindestens eines Diorganopolysiloxans mit einer Benzotriazolgruppe enthält, ausgewählt aus solchen der Formel:

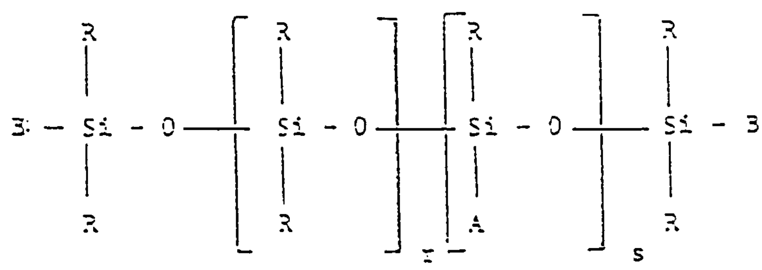 (1)

worin bedeuten:
R ist gleich oder verschieden und ausgewählt aus den $C_1$-$C_{10}$-Alkylradikalen, Phenyl und 3,3,3-Trifluorpropyl, wobei mindestens 80 % der Zahl der Radikale R Methylradikale sind,
B identisch oder gleich ist und ausgewählt ist aus den Radikalen R und dem Radikal A,
r ist eine Zahl ausgewählt zwischen 0 und einschließlich 200,
s ist eine Zahl ausgewählt zwischen 0 und einschließlich 50,
und wenn s 0 ist, ist mindestens einer der beiden Symbole B A,
und solchen der Formel:

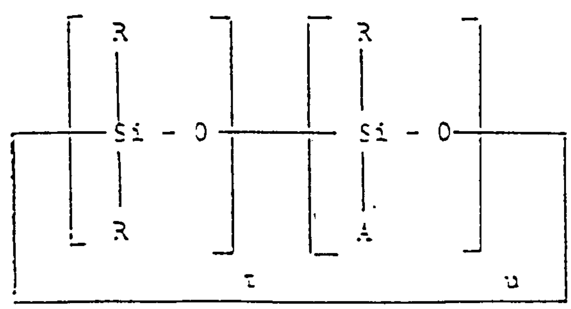 (2)

worin
R die gleiche Bedeutung wie in Formel (1) besitzt,
u ist eine Zahl zwischen 1 und einschließlich 20,
t ist eine Zahl zwischen 0 und einschließlich 20,
t + u ist gleich oder größer als 3,
und in denen das Symbol A ein Radikal der Formel ist:

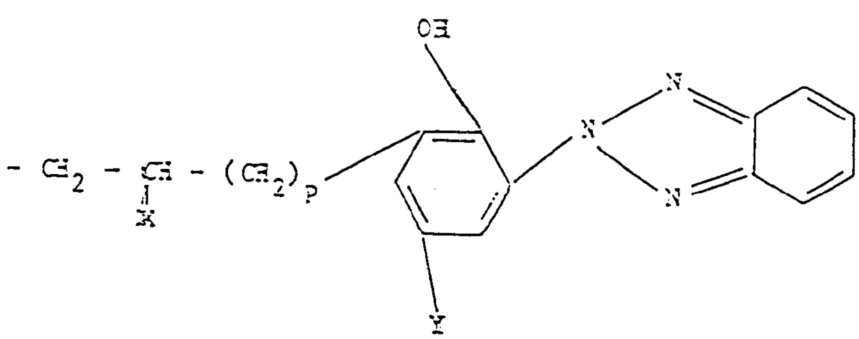 (3)

worin bedeuten:
X ist ein Wasserstoffatom oder ein $C_1$-$C_4$-Alkylradikal,
p ist eine ganze Zahl zwischen 1 und 10,
Y ist ein Wasserstoffatom oder ein $C_1$-$C_8$-Alkylradikal.

**8.** Kosmetische Zusammensetzung gemäß Anspruch 7, dadurch gekennzeichnet, daß sie ein Diorganopolysiloxan mit einer Benzotriazolgruppe der Formel (1) oder (2), als statistisches oder Block-Polymeres, enthält, das mindestens eine der folgenden Charakteristiken aufweist: R ist Methyl, B ist Methyl, r ist zwischen 5 und einschließlich 20, s ist zwischen 2 und einschließlich 15, t + u ist zwischen 3 und einschließlich 10, X ist H oder Methyl, p = 1 und Y ist Methyl.

9. Kosmetische Zusammensetzung nach Anspruch 7 oder 8, dadurch gekennzeichnet, daß sie ein Polydimethylsiloxan mit pfropfen aus 3-Allyl-2-hydroxy-5-methyl-phenyl-benzotriazol der Formel (1) gemäß Anspruch 7 umfaßt, worin R und B eine Methylgruppe ist, r = 5 und s = 5.

10. Kosmetische Zusammensetzung gemäß Anspruch 7 oder 8, dadurch gekennzeichnet, daß es ein Polydimethylsiloxan mit Pfropfen aus 3-Allyl-2-hydroxy-5-methyl-phenyl-benzotriazol der Formel (1) umfaßt, worin R und B Methyl bedeuten, r= 8 und s = 9.

11. Kosmetische Zusammensetzung gemäß einem der Ansprüche 7 bis 10, dadurch gekennzeichnet, daß sie außerdem kosmetische Additive enthält, ausgewählt aus Verdickungsmitteln, Weichmachern, Feuchthaltemitteln, oberflächenaktive Stoffen, Konservierungsmitteln, Antischaumstoffen, Parfüms, Ölen, Wachsen, Lanolin, niederen Monoalkoholen und Polyolen, Benzoaten der $C_{12}$-$C_{15}$-Alkohole, Treibmittel, Farbstoffe und Pigmente.

12. Kosmetische Zusammensetzung gemäß einem der Ansprüche 7 bis 11, dadurch gekennzeichnet, daß sie in Form einer öligen, alkoholischen oder oleoalkoholischen Lotion, einer Emulsion, eines öligen, oleoalkoholischen, alkoholischen oder hydroalkoholischen Gels, eines festen Stifts, eines Sprays oder Aerosols vorliegt.

13. Kosmetische Zusammensetzung nach einem der Ansprüche 7 bis 12, dadurch gekennzeichnet, daß sie eine Zusammensetzung zum Schutz der menschlichen Epidermis darstellt, die 0.25 bis 3 Gew.-% des Diorganopolysiloxans der Formel (1) oder (2) enthält.

14. Kosmetische Zusammensetzung nach einem der Ansprüche 7 bis 12 in Form einer Sonnenschutz-Zusammensetzung, dadurch gekennzeichnet, daß sie 0.5 bis 15 Gew.-% Diorganopolysiloxan der Formel (1) oder (2) enthält.

15. Kosmetische Sonnenschutz-Zusammensetzung gemäß Anspruch 14, dadurch gekennzeichnet, daß sie außerdem einen Filter für UV-B- und/oder UV-A-Strahlen enthält.

16. Kosmetische Zusammensetzung gemäß einem der Ansprüche 7 bis 11 zur Applikation auf Haaren, dadurch gekennzeichnet, daß sie in Form eines Shampoos, einer Lotion, eines Gels oder einer Spülemulsion vorliegt, zur Applikation vor oder nach dem Shampoonieren, vor oder nach dem Färben oder Entfärben, vor oder nach einer Dauerwellbehandlung, als Frisier- oder Formgebungslotion oder Gel, als Fön- oder Wasserwell-Lotion oder -Gel, als Frisierspray, Haarlack, und 0.25 bis 5 Gew.-% von Diorganopolysiloxan der Formel (1) oder (2) umfaßt.

17. Kosmetische Zusammensetzung gemäß einem der Ansprüche 7 bis 11 in Form einer gefärbten oder nichtgefärbten kosmetischen Zusammensetzung, dadurch gekennzeichnet, daß sie eine Haarzusammensetzung ist, ein Schminkprodukt oder eine Zusammensetzung zur Pflege und Behandlung der Haut, und 0.25 bis 3 Gew.-% Diorganopolysiloxan der Formel (1) oder (2) enthält.

18. Kosmetisches Verfahren zum Schutz der Haut und der natürlichen oder sensibilisierten Haare gegenüber ultravioletten Strahlen, dadurch gekennzeichnet, daß man auf die Haut oder die Haare eine wirksame Menge einer kosmetischen Zusammensetzung appliziert, die mindestens ein Diorganopolysiloxan mit einer Benzotriazol-Gruppe der Formel (1) oder (2), wie sie in einem der Ansprüche 3 bis 6 definiert ist, enthält.

19. Verfahren zum Schutz einer kosmetischen Zusammensetzung gegen ultraviolette Strahlung, dadurch gekennzeichnet, daß man in diese Zusammensetzung eine wirksame Menge von mindestens einem Diorganopolysiloxan mit einer Benzotriazolgruppe der Formel (1) oder (2), wie sie in einem der Ansprüche 3 bis 6 definiert sind, einbaut.